Europäisches Patentamt

(19)　European Patent Office

　　　Office européen des brevets

(11)　**EP 0 627 394 B1**

(12)　## EUROPEAN PATENT SPECIFICATION

(45) Date of publication and mention
of the grant of the patent:
**02.04.1997 Bulletin 1997/14**

(51) Int. Cl.$^6$: **C07C 5/27**, C07C 11/09,
B01J 29/04

(21) Application number: **94201383.0**

(22) Date of filing: **17.05.1994**

(54) **Process for the preparation of branched olefins**

Verfahren zur Herstellung von verzweigten Olefinen

Procédé pour la préparation d'olefines branchées

(84) Designated Contracting States:
**DE FR GB IT NL**

(30) Priority: **18.05.1993 EP 93201426**

(43) Date of publication of application:
**07.12.1994 Bulletin 1994/49**

(73) Proprietor: **SHELL INTERNATIONALE
RESEARCH
MAATSCHAPPIJ B.V.
2596 HR Den Haag (NL)**

(72) Inventors:
• **Kraushaar-Czarnetzki, Bettina
NL-1031 CM Amsterdam (NL)**
• **Jongkind, Hermanus
NL-1031 CM Amsterdam (NL)**

(56) References cited:
**EP-A- 0 132 708**　　　**EP-A- 0 161 489**
**EP-A- 0 523 838**　　　**US-A- 5 107 050**
**US-A- 5 132 484**

## Description

The present invention relates to a process for the preparation of branched olefins from a hydrocarbonaceous feedstock comprising linear olefins having at least four carbon atoms.

One of the main objects in nowaday's oil refining is to produce gasolines fulfilling the increasing environmental demands on product quality and having a high octane number.

This means for gasoline that the octane specification has now to be established without lead-containing additives, less aromatics, in particular benzene, less olefins and lower gasoline vapour pressure.

Components which are suitable for improving the octane quality of gasoline are certain highly branched ethers, e.g. methyl tertiary butyl ether (MTBE). It is known that such ethers can be formed by contacting branched olefins, e.g. branched butenes, with methanol in the presence of a suitable acidic catalyst, such as sulphonic resins, phosphoric acid, modified kieselguhr, silica/alumina and acidic zeolites.

A process for preparing branched butenes has for instance been described in US 5,146,035. In said disclosure it has i.a. been indicated that in a process for the isomerisation of butenes use can be made of a catalyst comprising a metal aluminophosphate as described in general terms in US 4,567,029.

Surprisingly, it has now been found that an improved yield of branched olefins can be obtained if a hydrocarbonaceous feedstock comprising linear olefins having at least four carbon atoms is contacted with a catalyst comprising a medium-pore molecular sieve selected from a specific group of MeAPO and MeAPSO medium-pore molecular sieves.

Accordingly, the present invention relates to a process for the preparation of branched olefins comprising contacting a hydrocarbonaceous feedstock comprising linear olefins having at least four carbon atoms at elevated temperature with a catalyst which comprises a MeAPO and/or MeAPSO medium-pore molecular sieve having an anhydrous composition expressed in molar oxide ratios as follows; $(MeO)_a(Al_2O_3)_b(P_2O_5)_c(SiO_2)_d$, whereby $(a + b)/c$ is greater than 1.0, a ranges from 0.003 to 0.2, b and c range from 0.05 to 0.3, d is at most 0.4 and $a/(c + d)$ is at least 0.05 when $SiO_2$ is present, and Me is at least one of Mg, Mn, Co and Zn.

In this way a very attractive high yield of branched olefins can be obtained.

In the context of the present invention a MeAPO medium-pore molecular sieve is defined as a crystalline microporous metal aluminophosphate composition having a three-dimensional framework structure of interconnected $MeO_2$, $AlO_2$ and $PO_2$ tetrahedral units.

This type of medium-pore molecular sieve and its preparation have for instance been described in US 4,567,029 which is hereby incorporated by reference.

Further, in the context of the present invention a MeAPSO medium-pore molecular sieve is defined as a crystalline microporous metal silicoaluminophosphate having a three-dimensional framework structure of interconnected $MeO_2$, $AlO_2$, $SiO_2$ and $PO_2$ tetrahedral units.

MeAPSO medium-pore molecular sieves and their preparation have for example been disclosed in EP 158348, EP 158975, EP 161489 and EP 161490 which are hereby incorporated by reference.

Preferably, in the process according to the present invention use is made of a MeAPO and/or MeAPSO medium-pore molecular sieve having an anhydrous composition expressed in molar oxide ratios as follows: $(MeO)_a(Al_2O_3)_b(P_2O_5)_c(SiO_2)_d$, wherein a ranges from preferably from 0.01 to 0.15, b ranges from 0.05 to 0.25, c ranges from 0.05 to 0.25 and d is at most 0.3.

Preferably, the hydrocarbonaceous feedstock comprising linear olefins having at least four carbon atoms is contacted with a catalyst comprising a MeAPO and/or MeAPSO medium-pore molecular sieve, wherein Me is at least Co.

Suitable MeAP(S)O catalysts include catalysts identified by the IUPAC Commission on zeolite nomenclature as AEL (CoAP(s)O-11), AFO, AFR, AFS, AFY, ATN and ATO. Preferred MeAP(S)O catalysts include CoAPO-11 type catalysts.

In the context of the present invention a medium-pore molecular sieve is defined as having an average pore diameter in the range of 3.8 to 7 Å, preferably in the range of 4 to 6.5 Å.

The catalyst to be used in the process according to the present invention may comprise one or more MeAPO and/or one or more MeAPSO medium-pore molecular sieves as defined hereinabove.

The catalyst may comprise in addition to the MeAPO and/or MeAPSO medium-pore molecular sieve a porous matrix material. Suitable porous matrix materials include, for instance, silica, alumina, zirconia and titania and mixtures thereof.

When $SiO_2$ is present in the medium-pore molecular sieve to be applied in the present process, $a/(c + d)$ is preferably at least 0.08.

Suitably, use is made in the present process of a medium-pore molecular sieve as described hereinbefore, wherein $(a + b)/c$ is at most 3.

Suitably, use is made in the present process of a medium-pore molecular sieve as described hereinbefore, wherein $a/(c + d)$ is at most 2.

The hydrocarbonaceous feedstock to be converted in accordance with the present invention comprises linear olefins having at least four carbon atoms.

In the context of the present invention, the hydrocarbonaceous feedstock may either comprise two or more types of linear olefins having at least four carbon atoms or may comprise only one specific type of linear olefin having at least four carbon atoms.

Suitably, the feedstock comprises linear olefins having four to ten carbon atoms. Preferably, the feedstock comprises linear olefins having four to six carbon atoms. More preferably, the feedstock comprises n-butene(s) and/or n-pentene(s). Suitably, the feedstock comprises at least 50 %wt n-butene(s) and/or n-pentene(s). Suitably, the feedstock consists essentially of n-butene(s).

Suitably, the hydrocarbonaceous feedstock to be converted has been obtained from a steam cracking and/or catalytic cracking process.

Process conditions which can suitably be applied, comprise a temperature of 200 to 480 °C and an olefin partial pressure of at least 0.5 bar and a total pressure of between 0.5 and 25 bar. Preferred conditions comprise a temperature of 300 to 450 °C and an olefin partial pressure of at least 0.7 bar.

Suitably, unconverted linear olefins are separated from branched olefins downstream the reaction zone wherein the isomerisation process is carried out, and recycled to the reaction zone. Such a separation can suitably be carried out by means of a molecular sieve.

The invention will now be illustrated by means of the following Examples.

Example 1 (according to the invention)

Catalyst preparation.

Cobalt (II) acetate tetrahydrate dissolved in water was combined with aluminium isopropoxide and homogenized until a gel was obtained. A second solution, consisting of orthophosphoric acid and water, and subsequently di-n-propylamine were added under agitation. The composition of the final reaction gel expressed in molar (oxide) ratios was as follows: 0.17 CoO : 1 $Al_2O_3$ : 1 $P_2O_5$ : 1 R : 45 $H_2O$, wherein R is di-n-propylamine. The reaction gel was then transferred to an autoclave to crystallize at 160 °C for 48 hours. The solid material obtained was subsequently recovered by filtration, washed with water and dried in air at 120 °C. Thereafter, the solid material was calcined in air at 550 °C for 3 hours. The product obtained had an X-ray diffraction pattern characteristic of highly crystalline CoAPO-11, and its anhydrous composition expressed in molar ratios was as follows: $(CoO)_{0.047}(Al_2O_3)_{0.27}(P_2O_2)_{0.25}$.

Subsequently, the CoAPO-11 powder obtained was pressed, crushed and sieved in order to obtain a 40-80 mesh fraction of pellets of a catalyst A.

Example 2 (according to the invention)

Catalyst preparation.

A catalyst B was prepared in the same way as catalyst A, except that the final reaction gel had a composition in molar (oxide) ratios as follows: 0.1 CoO : 1 $Al_2O_3$ : 1 $P_2O_5$ : 1 R : 45 $H_2O$, and the product obtained after calcination in air at 550 °C for 3 hours had the following anhydrous composition expressed in molar oxide ratios: $(CoO)_{0.024}(Al_2O_3)_{0.25}(P_2O_5)_{0.25}$.

Example 3 (not according to the invention)

Catalyst preparation.

A catalyst C was prepared in the same way as catalysts A and B, except that the final reaction gel had a composition in molar (oxide) ratios as follows: 0.05 CoO : 1 $Al_2O_3$ : 1 $P_2O_5$ : 1 R : 45 $H_2O$, and the product obtained after calcination in air at 550 °C for 3 hours had the following anhydrous composition expressed in molar oxide ratios: $(CoO)_{0.009}(Al_2O_3)_{0.24}(P_2O_5)_{0.25}$.

Example 4

Experiments.

Each of the catalysts A-C was contacted in the presence of argon with a feedstock consisting of 1-butene at a temperature of 350 °C, an olefin partial pressure of 1 bar, a total pressure of 4 bar, and a weight hourly space velocity of 2 kg/ (kg.h). The products obtained after 45 hours (catalysts A and C) or 46 hours (catalyst B) on stream are shown in Table 1 hereinbelow.

Table 1

| Catalyst composition: | A | B | C |
|---|---|---|---|
| (a + b)/c | 1.3 | 1.1 | 1.0 |
| Product (% by weight): | | | |
| ethene | | | 0.11 |
| propane | | 0.11 | 0.28 |
| propene | 0.8 | 1.98 | 3.53 |
| butane | 0.95 | 1.52 | 2.20 |
| n-butene | 41.51 | 31.52 | 27.24 |
| iso-butene | 37.89 | 30.85 | 26.65 |
| $C_5$+ liquids | 18.85 | 34.02 | 39.99 |

It will be clear from the above that processes which are carried out in accordance with the present invention (use of catalysts A and B) are much more attractive in terms of iso-butene production than a process just falling outside the scope of the present invention (use of catalyst C).

**Claims**

1. Process for the preparation of branched olefins comprising contacting a hydrocarbonaceous feedstock comprising linear olefins having at least four carbon atoms at elevated temperature with a catalyst which comprises a MeAPO and/or MeAPSO medium-pore molecular sieve having an anhydrous composition expressed in molar oxide ratios as follows; $(MeO)_a(Al_2O_3)_b(P_2O_5)_c(SiO_2)_d$, whereby (a + b)/c is greater than 1.0, a ranges from 0.003 to 0.2, b and c range from 0.05 to 0.3, d is at most 0.4 and a/(c + d) is at least 0.05 when $SiO_2$ is present, and Me is at least one of Mg, Mn, Co and Zn.

2. Process according to claim 1, wherein a ranges from 0.01 to 0.15, b and c range from 0.05 to 0.25, and d is at most 0.3 when $SiO_2$ is present.

3. Process according to claim 1 or 2, wherein Me is at least Co.

4. Process according to any one of claims 1-3, wherein a/(c + d) is at least 0.08 when $SiO_2$ is present.

5. Process according to any one of claims 1-4, wherein the hydrocarbonaceous feedstock comprises at least 50 %wt n-butene(s) and/or n-pentene(s).

6. Process according to any one of claims 1-5, wherein the process is carried out at a temperature of 250 to 480 °C, an olefin partial pressure of at least 0.5 bar and a total pressure of between 0.5 and 25 bar.

**Patentansprüche**

1. Verfahren zur Herstellung von verzweigten Olefinen, bei dem man einen geradkettige Olefine mit mindestens vier Kohlenstoffatomen enthaltenden kohlenwasserstoffhaltigen Einsatzstoff bei erhöhter Temperatur mit einem Katalysator, der ein MeAPO- und/oder MeAPSO-Molekularsieb mit mittlerer Porengröße enthält und eine wasserfreie Zusammensetzung, ausgedrückt in Form von Oxidmolverhältnissen, nämlich $(MeO)_a(Al_2O_3)_b(P_2O_5)_c(SiO_2)_d$, worin (a + b)/c größer als 1,0 ist, a im Bereich von 0,003 bis 0,2 liegt, b und c im Bereich von 0,05 bis 0,3 liegen, d höchstens 0,4 und a/(c + d) mindestens 0,05 beträgt, falls $SiO_2$ vorhanden ist, und Me mindestens ein Mitglied der Reihe Mg, Mn, Co und Zn bedeutet, aufweist, in Berührung bringt.

2. Verfahren nach Anspruch 1, bei dem a im Bereich von 0,01 bis 0,15 liegt, b und c im Bereich von 0,05 bis 0,25 liegen und d höchstens 0,3 beträgt, falls $SiO_2$ vorhanden ist.

3. Verfahren nach Anspruch 1 oder 2, bei dem Me mindestens Co bedeutet.

4. Verfahren nach einem der Ansprüche 1-3, bei dem

der a/(c + d) mindestens 0,08 beträgt, falls $SiO_2$ vorhanden ist.

5. Verfahren nach einem der Ansprüche 1-4, bei dem der kohlenwasserstoffhaltige Einsatzstoff mindestens 50 Gew.-% n-Buten(e) und/oder n-Penten(e) enthält.

6. Verfahren nach einem der Ansprüche 1-5, das man bei einer Temperatur von 250 bis 480°C, einem Olefinpartialdruck von mindestens 0,5 bar und einem Gesamtdruck zwischen 0,5 und 25 bar durchführt.

**Revendications**

1. Procédé de préparation d'oléfines ramifiées, comprenant la mise en contact d'une matière première hydrocarbonée comprenant des oléfines linéaires comportant au moins quatre atomes de carbone, à une température élevée, avec un catalyseur comprenant un tamis moléculaire à pores moyens de type MeAPO et/ou MeAPSO ayant la composition anhydre, exprimée en rapport molaire des oxydes, suivante: $(MeO)_a(Al_2O_3)_b(P_2O_5)_c(SiO_2)_d$, où (a + b)/c est supérieur à 1,0, a s'échelonne de 0,003 à 0,2, b et c s'échelonnent de 0,05 à 0,3, d est d'au plus 0,4 et a/(c + d) est d'au moins 0,05 lorsque $SiO_2$ est présent, et Me est au moins un élément parmi Mg, Mn, Co et Zn.

2. Procédé selon la revendication 1, dans lequel a s'échelonne de 0,01 à 0,15, b et c s'échelonnent de 0,05 à 0,25, et d est d'au plus 0,3 lorsque $SiO_2$ est présent.

3. Procédé selon la revendication 1 ou 2, dans lequel Me est au moins Co.

4. Procédé selon l'une quelconque des revendications 1-3, dans lequel a/(c + d) est d'au moins 0,08 lorsque $SiO_2$ est présent.

5. Procédé selon l'une quelconque des revendications 1-4, dans lequel la matière première hydrocarbonée comprend au moins 50% en poids de n-butène(s) et/ou de n-pentène(s).

6. Procédé selon l'une quelconque des revendications 1-5, dans lequel le procédé est réalisé à une température de 250 à 480°C, sous une pression partielle d'oléfine d'au moins 0,5 bar et sous une pression totale comprise entre 0,5 et 25 bars.